# DEMANDE DE BREVET EUROPEEN

(11) **EP 1 306 081 A1**
(43) Date de publication de la demande: **02.05.2003**
(21) Numéro de dépôt: 02292612.5
(22) Date de dépôt: 22.10.2002
(51) Int. Cl.: A61K 7/48

(54) **Utilisation cosmétique de dérivés de la DHEA**

(30) Priorité: 25.10.2001 FR 0113817
(71) Demandeur: L'OREAL, 75008 Paris (FR)
(72) Inventeur: Dalko, Maria, 91190 GIF S/Yvette (FR); Cavezza, Alexandre, 93290 Tremblay-en-France (FR)
(74) Mandataire: Renard, Emmanuelle

(57) **Abrégé**

L'invention se rapporte à l'utilisation cosmétique d'au moins un dérivé de la DHEA pour améliorer l'apparence des matières kératiniques, tels que la peau, les cheveux, les cils et les ongles, en particulier pour prévenir ou traiter les signes cutanés du vieillissement et/ou le teint terne et/ou les troubles de pigmentation de la peau ou des cheveux et/ou le dessèchement de la peau et/ou l'hyperséborrhée et/ou les imperfections liées à l'hyperséborrhée et/ou les peaux sensibles et/ou les pellicules et/ou la chute des cheveux et/ou la canitie.

L'invention se rapporte également à un procédé de traitement cosmétique des matières kératiniques comprenant l'application topique sur celles-ci d'une composition renfermant au moins un dérivé de la DHEA dans un milieu physiologiquement acceptable.

## Description

L'invention se rapporte à l'utilisation cosmétique d'au moins un dérivé de la DHEA pour améliorer l'apparence des matières kératiniques, tels que la peau, les cheveux, les cils et les ongles, en particulier pour prévenir ou traiter les signes cutanés du vieillissement et/ou le teint terne et/ou les troubles de pigmentation de la peau ou des cheveux et/ou le dessèchement de la peau et/ou l'hyperséborrhée et/ou les imperfections liées à l'hyperséborrhée et/ou les peaux sensibles et/ou les pellicules et/ou la chute des cheveux et/ou la canitie.

L'invention se rapporte également à un procédé de traitement cosmétique des matières kératiniques comprenant l'application topique sur celles-ci d'une composition renfermant au moins un dérivé de la DHEA dans un milieu physiologiquement acceptable.

La DHEA, ou déhydroépiandrostérone, est un stéroïde naturel produit essentiellement par les glandes corticosurrénales. La DHEA exogène, administrée par voie topique ou orale, est connue pour sa capacité à promouvoir la kératinisation de l'épiderme (JP-07 196 467) et à traiter les peaux sèches en augmentant la production endogène et la sécrétion de sébum et en renforçant ainsi l'effet barrière de la peau (US-4,496,556). Il a également été décrit dans le brevet US-5,843,932 l'utilisation de la DHEA pour remédier à l'atrophie du derme par inhibition de la perte de collagène et de tissu conjonctif. Enfin, la Demanderesse a mis en évidence la capacité de la DHEA à lutter contre l'aspect papyracé de la peau (FR 00/00349), à moduler la pigmentation de la peau et des cheveux (FR 99/12773) et à lutter contre l'atrophie de l'épiderme (FR 00/06154). Ces propriétés de la DHEA en font un candidat de choix comme actif anti-âge.

Toutefois, la DHEA présente des effets de nature hormonale pouvant rendre son utilisation délicate.

De ce fait, on comprend que l'on ait cherché à disposer de dérivés de la DHEA présentant des propriétés cosmétiques aussi intéressantes que la DHEA elle-même, mais n'ayant pas d'effets hormonaux.

Il est apparu à la Demanderesse que les dérivés de la DHEA de formule générale (I) suivante pouvaient permettre de répondre à ce besoin :

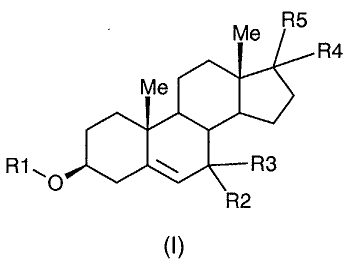

Parmi les dérivés de la DHEA, on connaît déjà la 3β-acétoxy-7-oxo-DHEA ou Δ5-androstène-3β-acétoxy-7,17-dione, qui a été décrite comme étant efficace dans la modulation du système immunitaire (US-5,292,730 ; US-5,585,371 ; US-5,641,766), le traitement de la maladie d'Alzheimer (US-5,707,983) et le traitement du syndrome HIV (US-5,885,977) et pour favoriser la perte de poids (US-5,296,481 ; US-5,807,848).

Le document WO 99/25333 mentionne en outre l'utilisation, notamment topique, de la 3β-acétoxy-7-oxo-DHEA dans le traitement prophylactique et curatif du lupus érythémateux, qui est un trouble du système immunitaire susceptible d'affecter plusieurs organes et se manifestant, au niveau de la peau, par une rougeur transversale de la face et/ou par des plaques d'érythème squameux disséminées sur le corps.

Le document US 5,424,463 décrit notamment la 7-céto-DHEA (ou Δ5-androstène-3β-ol-7,17-dione) ainsi que ses dérivés hydrolysables obtenus par modification du groupe 3β-hydroxy, lesdits dérivés étant susceptibles de redonner la 7-céto-DHEA après hydrolyse. Le groupe 3β-hydroxy est transformé en carbamate ou est estérifié par (i) un acide aliphatique en C2-C22, normal, branché, saturé ou insaturé, (ii) un acide aromatique en C7-C22, (iii) un acide dicarboxylique en C3 ou plus, pour lequel seul un groupement carbonyl est estérifié avec le groupe 3-hydroxy du stéroïde, (iv) un acide inorganique tel que l'acide sulfurique et l'acide phosphorique. Le document US 5,424,463 décrit la 7-céto-DHEA et ses dérivés hydrolysables comme étant efficaces pour favoriser la perte de poids.

Le document WO98/40074 décrit l'utilisation de dérivés de la DHEA de formule (A) : 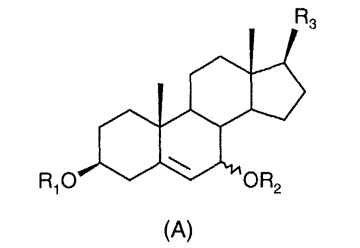 dans laquelle :
- R₁ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbones de 3 à 100 atomes de carbone et leurs dérivés comprenant ou non un ou plusieurs atomes d'azote ;
- R2 est choisi parmi : un atome d'hydrogène ou une fonction ester d'acide gras de 1 à 24 atomes de carbone ;
- R3 est choisi parmi : un atome d'hydrogène, un groupe -OH, les groupes de formules : -CO-R₄, -CHOH-R₄, =CH-CH₃, =COH-CH₃, -CHR₄-CH₃, =O, dans lesquels R₄ est un groupe alcoyle comprenant de 1 à 10 atomes de carbone substitué ou non ;
dans une composition pour prévenir ou traiter les manifestations du vieillissement cutané et/ou les effets d'irradiations UV sur la peau.

On connaît encore le document WO00/28996 qui décrit une composition pour améliorer la texture des tissus, comprenant un dérivé de la DHEA de formule (A) en association avec un peptide dérivé de l'élastine obtenu par clivage sélectif de l'élastine par la thermolysine.

Toutefois, à la connaissance de la Demanderesse, il n'a encore jamais été proposé d'utiliser les dérivés de la DHEA de formule générale (I) à des fins cosmétiques, en particulier dans le traitement des signes du vieillissement.

L'invention a donc pour objet l'utilisation cosmétique, pour améliorer l'apparence des matières kératiniques, d'au moins un dérivé de la DHEA de formule (I) suivante : 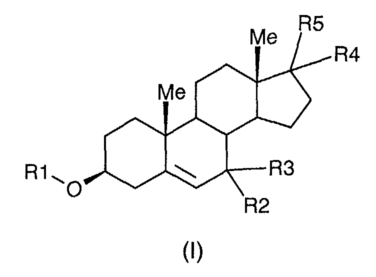 dans laquelle :
**R1** est choisi parmi :
   - un atome d'hydrogène ;
   - un groupe alkyle en C1-C12, saturé ou insaturé, linéaire ou ramifié, cyclique pouvant éventuellement contenir un ou plusieurs hétéroatomes, et éventuellement substitué par un ou plusieurs groupes choisis parmi -OR' et/ou -SR' et/ou -COOR' et/ou -NR'R' et/ou halogène et/ou perfluoroalkyle et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
   - un groupe alkylcarbonyle, dont la partie alkyle en C1-C24 est saturée ou insaturée, linéaire ou ramifiée, cyclique, et éventuellement substituée par un ou plusieurs groupes choisi parmi -OR' et/ou -SR' et/ou -COOR' et/ou -NR'R' et/ou halogène et/ou perfluoroalkyle et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
   - un groupe phényle, éventuellement fonctionnalisé par un ou plusieurs groupes -OR' et/ou -SR' et/ou -COOR' et/ou -NR'R' et/ou halogène et/ou perfluoroalkyle et/ou sulfate et/ou phosphate et/ou aryle et/ou hétérocycle ;
   - un groupe benzyle, éventuellement fonctionnalisé par un ou plusieurs groupes -OR' et/ou -SR' et/ou -COOR' et/ou -NR'R' et/ou halogène et/ou perfluoroalkyle et/ou sulfate et/ou phosphate et/ou aryle et/ou hétérocycle ;
   - un groupe arylcarbonyle, de préférence un phénylcarbonyle, ou un groupe arylalkylcarbonyle, de préférence un benzylcarbonyle, éventuellement substitué par un ou plusieurs groupes -OR' et/ou -SR' et/ou -COOR' et/ou -NR'R' et/ou halogène et/ou perfluoroalkyle et/ou sulfate et/ou phosphate et/ou aryle et/ou hétérocycle ;
   - un groupe O=P(OH)OR' ;
   - un groupe (O)₂SOR' ;
   - un groupe (O)₂SR' ;
   - un groupe trialkylsilyle (SiR'₃) dans lequel les 3 groupes R' peuvent être identiques ou différents ;
   - un groupe alkyloxycarbonyle (R'OCO) ;
   - un groupe alkylaminocarbonyle (R'NR'''CO) ;
   - un hydrate de carbone de 3 à 100 atomes de carbones et leurs dérivés ;
**R4** et **R5** représentent :
   - ensemble un groupe choisi parmi :
      - un groupe céto (=O) ;
      - un groupe =CHR' ;
      - un groupe =NR' ;
      - un groupe =NCOR' ;
   - chacun un groupe -OR" identique, avantageusement les deux groupes -OR" forment ensemble un cycle 1,3-dioxolane ou 1,3-dioxane ;
   - chacun un groupe identique ou différent choisi parmi :
      - un atome d'hydrogène ;
      - un groupe -NR'R' ;
      - un groupe -NHCOR' ;
Lorsque **R3** représente un atome d'hydrogène, **R2** est choisi parmi :
   - un groupe -OR6 dans lequel R6 a les mêmes définitions que celles données précédemment pour R1 ;
   - un groupe amine -N(R7)₂ dans lequel les substituants R7, identiques ou différents, ont les mêmes définitions que celles données précédemment pour R1;
   - un groupe N-sulfonamide (-NR'''SO₂R') ou N-sulfonate (-NR'''SO₃R'), de préférence sous forme de sel de métal alcalin ;
   - un groupe urée (-NR'''CONR'R') ;
   - un groupe alkylamide (-NR'''COR') ;
   - un groupe N-carbamate (-NR'''COOR') ;
   - un groupe thiol (-SR') ;
   - un groupe thiophényl (-SPh) ;
   - un groupe sulfone (-SO₂R') ;
   - un groupe sulfoxide (-SOR') ;
   - un halogène ;
   - un groupe alkylsilane (-SiR'₃) dans lequel les 3 groupes R' peuvent être identiques ou différents ;
**R2** et **R3** peuvent également représenter ensemble un groupe choisi parmi :
   - un méthylène (=CHR') ;
   - une imine (=NHR') ;
   - une oxime (=NOR') ;
   - une hydrazone (=N-NR'R')
   - un groupe céto (=O) ;
**R2** et **R**3 peuvent encore représenter chacun un groupe -OR" dans lequel chacun des R" représente des chaînes alkyles en C1-C6, de préférence en C1-C3 et formant ensemble avantageusement un cycle de préférence à 5 chaînons (cycle 1,3-dioxolane) ou 6 chaînons (cycle 1,3-dioxane) ;
**R'** est choisi parmi un atome d'hydrogène, un groupe alkyle en C1-C12, de préférence en C1-C6, saturé ou insaturé, linéaire ou ramifié, cyclique pouvant éventuellement contenir un ou plusieurs hétéroatomes, éventuellement fonctionnalisé par un ou plusieurs groupes -OR"', -COOR''', halogène, -NR"'R"'; ou par un groupe aryle, de préférence un phényle, éventuellement fonctionnalisé par un ou plusieurs groupes -OR''', -COOR"', halogène ou -NR"'R"' ;de préférence R' représente un atome d'hydrogène, un méthyle, un éthyle, un butyle, un propyle, un isopropyle, un *tert*-butyle ;
**R'''** représente un atome d'hydrogène, une chaîne alkyle, de préférence en C1-C6, saturée ou insaturée, linéaires ou ramifiées ou cycliques ; de préférence R''' représente un atome d'hydrogène, un méthyle, un éthyle, un butyle, un propyle, un isopropyle, un *tert*-butyle ;
étant entendu que dans chacun des groupes -NR'R' et -NR"'R"', les substituants R', respectivement R''', sont identiques ou différents ;
à l'exception des dérivés de la DHEA de formule (I) pour lesquels :
- R2 et R3 représentent ensemble un groupe céto ;
- R4 et R5 représentent ensemble un groupe céto, et
- R1 est tel que défini précédemment ;
à l'exception des dérivés de la DHEA de formule (A) 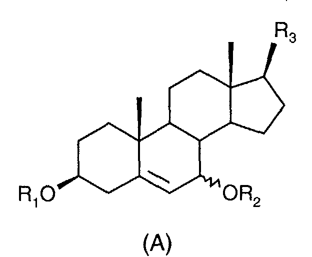 dans laquelle :
- R₁ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbones de 3 à 100 atomes de carbone et leurs dérivés comprenant ou non un ou plusieurs atomes d'azote ;
- R2 est choisi parmi : un atome d'hydrogène ou une fonction ester d'acide gras de 1 à 24 atomes de carbone ;
- R3 est choisi parmi : un atome d'hydrogène, un groupe -OH, les groupes de formules : -CO-R₄, -CHOH-R₄, =CH-CH₃, =COH-CH₃, -CHR₄-CH₃, =O, dans lesquels R₄ est un groupe alcoyle comprenant de 1 à 10 atomes de carbone substitué ou non.

L'invention concerne également les isomères optiques et/ou géométriques des dérivés de la DHEA de formule (I) seuls ou en mélange en toutes proportions, ainsi que les sels physiologiquement acceptables de ces dérivés.

Par "matières kératiniques", on entend de préférence la peau, les fibres capillaires (cheveux et cils) et les ongles.

Selon une forme préférée de l'invention, les dérivés de la DHEA de formule (I) préférés sont ceux pour lesquels **R1** représente un groupe alkyle en C1-C6, saturée ou insaturée, linéaire ou ramifié, ou cyclique pouvant contenir un ou plusieurs hétéroatomes, et éventuellement substitué par un ou plusieurs groupes choisis parmi -OR' et/ou -SR' et/ou -COOR' et/ou -NR'R' et/ou halogène.

Selon une autre forme préférée de l'invention, les dérivés de la DHEA de formule (I) préférés sont ceux pour lesquels **R1** représente un groupe alkylcarbonyle, dont la partie alkyle en C1-C20, de préférence en C6-C18, est saturée ou insaturée, linéaire ou ramifié ou cyclique et éventuellement substituée par un ou plusieurs groupes choisis parmi -OR' et/ou -SR' et/ou -COOR' et/ou -NR'R' et/ou halogène.

Lorsque **R3** représente un atome d'hydrogène, **R2** est choisi parmi :
- un groupe -OR6 dans lequel R6 a les mêmes définitions que celles données précédemment de manière préférée pour R1 ;
- un groupe amine -N(R7)₂ dans lequel les substituants R7, identiques ou différents, ont les mêmes définitions que celles données précédemment de manière préférée pour R1;
- un groupe thiol (-SR') ;
- un halogène.

Selon une autre forme préférée de l'invention, les dérivés de la DHEA de formule (I) préférés sont ceux pour lesquels chacun des groupes -NR'R' et/ou -NR"'R"' représente un acide aminé, de préférence choisi parmi la L-alanine, la L-arginine, la L-asparagine, acide L-aspartique, la L-cystéine, la L-glutamine, acide L-glutamique, la glycine, la L-histidine, la L-isoleucine, la L-leucine, la L-lysine, la L-méthionine, la L-phénylalanine, la L-proline, la L-sérine, la L-thréonine, le L-tryptophane, la L-tyrosine, la L-valine ;

Parmi les dérivés de formule (I), utilisés selon l'invention, on préfère tout particulièrement les composés suivants :
- Androst-5-en-17-one, 3-(acetyloxy)-7-(benzoyloxy)-, (3β,7α) ;
- Androst-5-en-17-one, 7-hydroxy-3-[[(1-oxopentyl)sulfonyl]oxy]-, (3β,7β) ;
- Androst-5-en-17-one, 3-hydroxy-7-(1-oxo-3-phenylpropoxy)-, (3β,7β) ;
- Androst-5-en-17-one, 7-hydroxy-3-[[3-(4-hydroxy-3-methoxyphenyl)-1-oxo-2-propenyl]oxy]-, (3β) ;
- Androst-5-en-17-one, 7-hydroxy-3-[[(9Z)-1-oxo-9-octadecenyl]oxy]-, (3β) ;
- Pregn-5-en-20-one, 3-(acetyloxy)-7-hydroxy-, (3β,7α) ;
- Butanoic acid, 4-[[[(3β,7Z)-3-hydroxy-17-oxoandrost-5-en-7-ylidene]amino]oxy] ;
- Butanoic acid, 4-[[[(3β,7Z)-3-(acetyloxy)-17-oxoandrost-5-en-7-ylidene]amino]oxy]-, methyl ester ;
- Androst-5-en-17-one, 3-(benzoyloxy)-7-(2-methyl-1-oxopropoxy)-, (3β,7β) ;
- Androst-5-en-17-one, 3-(acetyloxy)-7-fluoro-, (3β,7β) ;
- Androst-5-en-17-one, 3-(acetyloxy)-7-fluoro-, (3β,7α) ;
- Acetic acid, [[[(3β,7Z)-3-hydroxy-20-oxopregn-5-en-7-ylidene]amino]oxy] ;
- Acetic acid, [[[(3β,7Z)-3-(acetyloxy)-20-oxopregn-5-en-7-ylidene]amino]oxy]-, methyl ester ;
- Androst-5-en-17-one, 3-(acetyloxy)-7-bromo-, (3β) ;
- Androst-5-en-17-one, 7-bromo-3-hydroxy-, (3β,7α) ;
- Androst-5-en-17-one, 7-bromo-3-hydroxy-, (3β) ;
- Pregn-5-en-20-one, 7-bromo-3-hydroxy-, acétate ;
- Pregn-5-en-20-one, 3-(acetyloxy)-7-bromo-, (3β)
- Pregn-5-en-20-one, 7α-chloro-3β-hydroxy-, acétate ;
- Pregn-5-en-20-one, 3-(acetyloxy)-7-bromo-, (3β,7β) ;
- Pregn-5-en-20-one, 3-(acetyloxy)-7-bromo-, (3β,7α) ;
- Pregn-5-en-20-one, 7-bromo-3-hydroxy-, benzoate ;
- Pregn-5-en-20-one, 7-bromo-3β-hydroxy-, 4-methylvalerate ;
- Androst-5-en-17-one, 3β,7α-dihydroxy-, disulfate ;
- Acetic acid, [[(3β,7α)-3-hydroxy-17-oxoandrost-5-en-7-yl]thio] ;
- Androst-5-ene-7,17-dione, 3-(acetyloxy)-, 7,17-dioxime, (3β) ;
- Androst-5-en-17-one, 3-(benzoyloxy)-7-bromo-, (3β,7α) ;
- Acetic acid, [[[(3β,7Z)-3-hydroxy-17-oxoandrost-5-en-7-ylidene]amino]oxy] ;
- Acetic acid, [[[(3β,7Z)-3-hydroxy-17-oxoandrost-5-en-7-ylidene]amino]oxy]-, methyl ester ;
- Acetic acid, [[[(3β,7Z)-3-(acetyloxy)-17-oxoandrost-5-en-7-ylidene]amino]oxy]-, methyl ester ;
- Androst-5-en-17-one, 3,7-bis[(trimethylsilyl)oxy]-, (3β,7β) ;
- Androst-5-en-17-one, 7-hydroxy-3-[[(3-methylphenyl)sulfonyl]oxy]-, (3β,7α) ;
- Androst-5-en-17-one, 3-[(3-chlorobenzoyl)oxy]-7-hydroxy-, (3β,7α) ;
- Androst-5-en-17-one, 7-(3-carboxy-1-oxopropoxy)-3-[(1-oxoheptyl)oxy]-, (3β,7α) ;
- Pregn-5-en-20-one, 3,7-bis[(trimethylsilyl)oxy]-, (3β,7α) ;
- Androst-5-en-17-one, 3,7-bis[(trimethylsilyl)oxy]-, (3β,7α) ;
- Acetic acid, [[[(3β)-3-hydroxy-17-oxoandrost-5-en-7-ylidene]amino]oxy] ;
- Androst-5-en-17-one, 3-(acetyloxy)-7-methoxy-, (3β,7α) ;
- Androst-5-en-17-one, 3-(acetyloxy)-7-methoxy-, (3β,7β) ;
- Pregna-5,16-diene-7,20-dione, 3-(acetyloxy)-, 7,20-dioxime, (3β) ;
- Androst-5-en-17-one, 3-(acetyloxy)-7-bromo-, (3β,7α) ;
- Pregn-5-en-20-one, 3β,7α-dihydroxy-, 7-(hydrogen sulfate) ;
- Androst-5-en-17-one, 3-hydroxy-7-(sulfooxy)-, (3β,7α) ;
- Pregn-5-ene-7,20-dione, 3-hydroxy-, bis[(aminoiminomethyl)hydrazone], (3β) ;
- Guanidine, 1,1'-[(3β-hydroxypregn-5-ene-7,20-diylidene)dinitrilo]di-, dihydrochloride ;
- Androst-5-en-17-one, 7-bromo-3β-hydroxy-, benzoate ;
- Androst-5-en-17-one, 3-hydroxy-7-methoxy-, (3β,7α) ;
- Androst-5-en-17-one, 3-hydroxy-7-methoxy-, (3β,7β) ;
- Androst-5-en-7-one, 3-(acetyloxy)-17-(benzoyloxy)-, 7-[O-(phenylmethyl)oxime], (3β,7E,17β) ;
- Androst-5-ene-3,7,17-triol, 17-(hydrogen sulfate), (3β,7β,17β) ;
- Androst-5-en-7-one, 3,17-bis(acetyloxy)-, 7-[O-(3-hydroxypropyl)oxime], (3β,7Z,17β) ;
- Propanoic acid, 3-[[[(3β,7Z,17β)-3,17-bis(acetyloxy)androst-5-en-7-ylidene]amino]oxy]-, methyl ester ;
- Propanoic acid, 3-[[[(3β,7Z,17β)-3,17-bis(acetyloxy)androst-5-en-7-ylidene]amino]oxy] ;
- Androst-5-en-17-one, 7-(acetyloxy)-3-[[(1,1-dimethylethyl)dimethylsilyl]oxy]-, cyclic 17-(1,2-ethanediyl acetal), (3β,7β) ;
- Androst-5-en-7-one, 3-(acetyloxy)-17-[[(1,1-dimethylethyl)dimethylsilyl]oxy]-, cyclic 7-(1,2-ethanediyl acetal), (3β,17β) ;
- Androst-5-ene-7,17-diol, 3-[[(1,1-dimethylethyl)diphenylsilyl]oxy]-, 17-octanoate, (3β,7β,17β) ;
- Butanoic acid, 4-[[[(3β,7Z,17β)-3-(acetyloxy)-17-hydroxyandrost-5-en-7-ylidene]amino]oxy]-, methyl ester ;
- Androst-5-ene-3,7,17-triol, 17-acetate 3-benzoate, (3β,7β,17β) ;
- Acetic acid, [[[(3β,7Z,17β)-3-(acetyloxy)-17-hydroxyandrost-5-en-7-ylidene]amino]oxy]-, methyl ester ;
- Androst-5-ene-3,7,17-triol, 3,17-diacetate 7-methanesulfonate, (3β,17β) ;
- Androst-5-ene-3,17-diol, 7-bromo-, diacetate, (3β,7α,17β) ;
- Androst-5-ene-3,7,17-triol, 3-acetate 17-benzoate, (3β,7α,17β) ;
- Androst-5-ene-3,7,17-triol, 3-acetate 17-benzoate, (3β,7β,17β) ;
- Androst-5-ene-3,17-diol, 7-bromo-, (3β,7α,17β) ;
- Androst-5-ene-3,17-diol, 7-bromo-, (3β,17β) ;
- Androst-5-ene-3,17-diol, 7-bromo-, 3-acetate 17-benzoate, (3β,7α,17β) ;
- Androst-5-ene-3,17-diol, 7-(phenylsulfinyl)-, diacetate, (3β,17β) ;
- Androst-5-ene-3,17-diol, 7-(phenylthio)-, diacetate, (3β,17β) ;
- Androst-5-ene-3,17-diol, 7-bromo-, diacetate, (3β,17β) ;
- Androst-5-ene-3β,17β-diol, 7-bromo-, dibenzoate ;
- Androst-5-ene-3,17-diol, 7-bromo-, 3-acetate 17-benzoate, (3β,7β,17β) ;
- Propanoic acid, 3-[[(3β,7α,17β)-3-(acetyloxy)-17-(benzoyloxy)androst-5-en-7-yl]thio]-, methyl ester ;
- Propanoic acid, 3-[[(3β,7α,17β)-3,17-dihydroxyandrost-5-en-7-yl]thio] ;
- Androst-5-en-7-one, 3-(acetyloxy)-17-(benzoyloxy)-, 7-[O-(phenylmethyl)oxime], (3β,7Z,17β) ;
- Androst-5-ene-3,7,17-triol, 3-acetate 7,17-dibenzoate, (3β,7α,17β) ;
- Androst-5-ene-3,7,17-triol, 3-acetate 7,17-dibenzoate, (3β,7β,17β) ;
- Androst-5-en-17-one, 3-(acetyloxy)-7-bromo-, cyclic 1,2-ethanediyl acetal, (3β,7β) ;
- Androst-5-en-7-one, 3-(acetyloxy)-17-(benzoyloxy)-, 7-[O-(phenylmethyl)oxime], (3β,17β) ;
- Androst-5-en-17-one, 3,7-bis(acetyloxy)-, cyclic 17-(1,2-ethanediyl acetal), (3β,7α) ;
- Androst-5-en-17-one, 3-(acetyloxy)-7-hydroxy-, cyclic 17-(1,2-ethanediyl acetal), (3β,7β) ;
- Androst-5-en-17-one, 3-(acetyloxy)-7-hydroxy-, cyclic 17-(1,2-ethanediyl acetal), (3β,7α) ;
- Androst-5-ene-7,17-dione, 3-(acetyloxy)-, 7,17-dioxime, (3β) ;
- Androst-5-en-3-ol, 7-bromo-, benzoate, (3β) ;
- Androst-5-ene-7,17-dione, 3-(acetyloxy)-, 17-(1,2-ethanediyl acetal), 7-oxime, (3β,7Z) ;
- Acetic acid, [[[(3β,7Z)-3-(acetyloxy)-17,17-[1,2-ethanediylbis(oxy)]androst-5-en-7-ylidene]amino]oxy]-, methyl ester ;
- Androst-5-en-7-one, 3,17-bis[(trimethylsilyl)oxy]-, O-methyloxime, (3β,17β) ;
- Androst-5-en-7-one, 3,17-dihydroxy-, oxime, (3β,17β) ;
- Androst-5-ene-7,17-dione, 3-(acetyloxy)-17 oxime (3β) ;
- Androst-5-ene-7,17-dione -3-(acetyloxy)-7,17dioxime (3β) ;
- Acetic acid, [[[(3β,17β)-3,17-dihydroxyandrost-5-en-7-ylidene]amino]oxy] ;
- Acetic acid, [[[(3β)-17,17-[1,2-ethanediylbis(oxy)]-3-hydroxyandrost-5-en-7-ylidene]amino]oxy] ;
- Silane, [[(3β,7α,17β)-androst-5-ene-3,7,17-triyl]tris(oxy)]tris[trimethyl] ;
- Benzenesulfonic acid, 4-methyl-, [(3β,17β)-3,17-bis(acetyloxy)androst-5-en-7-ylidene]hydrazide ;
- Androst-5-en-7-one, 3,17-bis(acetyloxy)-, oxime, (3β,17β) ;
- Androst-5-en-17-one, 3-(acetyloxy)-7-bromo-, cyclic 1,2-ethanediyl acetal, (3β,7α) ;
- Androst-5-en-17-one, 7-bromo-3-hydroxy-, cyclic 1,2-ethanediyl acetal, (3β,7α) ;
- Androst-5-en-3β-ol, 7α-bromo-, acétate ;
- Androst-5-en-3β-ol, 7α-bromo.

Les dérivés de la DHEA de formule (I) selon l'invention sont facilement accessibles d'un point de vue synthétique, notamment par la mise en oeuvre d'une des méthodes de synthèse décrites dans les brevets US-5,424,463 ; FR-2 771 105 ; US-5,869,709 ; US-6,111,118, WO-94/085888, WO01/23405 et/ou dans le document Tetrahedron Letters, 1997, 38, 119-122

Plus particulièrement, l'invention se rapporte à l'utilisation cosmétique d'au moins un dérivé de la DHEA de formule (I) tel que défini précédemment pour prévenir ou traiter les signes cutanés du vieillissement et/ou le teint terne et/ou les troubles de pigmentation de la peau ou des cheveux et/ou le dessèchement de la peau et/ou l'hyperséborrhée et/ou les imperfections liées à l'hyperséborrhée et/ou les peaux sensibles et/ou les pellicules et/ou la chute des cheveux et/ou la canitie.

Par "signes cutanés du vieillissement", on entend : les rides et ridules, la perte de fermeté et/ou d'élasticité de la peau, l'atrophie cutanée, un grain de peau plus irrégulier avec présence de pores dilatés, la perte d'éclat de la peau et/ou les taches pigmentaires.

Par "peaux sensibles", on entend les peaux qui ont été caractérisées par la Demanderesse dans le brevet EP 0 680 749 B1. La Demanderesse a pu montrer que les symptômes liés aux peaux sensibles étaient constitués de sensations plus ou moins douloureuses ressenties dans une zone cutanée, tels que des picotements, fourmillements, démangeaisons ou prurits, brûlures, rougeurs, échauffements, inconforts, tiraillements, etc. Ces symptômes peuvent se manifester en réponse à différents facteurs tels que entre autres, la sueur, les frottements, les émotions, les aliments, le vent, le rasoir, le savon, les tensioactifs, l'eau dure à forte concentration de calcaire, les variations de température ou la laine.

Un autre objet de la présente invention se rapporte à une composition cosmétique renfermant, dans un milieu physiologiquement acceptable, au moins un dérivé de la DHEA de formule (I) tel que défini précédemment et au moins un composé choisi parmi : un agent desquamant, un agent hydratant, un agent dépigmentant ou propigmentant, un agent anti-glycation, un inhibiteur de NO-synthase, un inhibiteur de 5α-réductase, un inhibiteur de lysyl et/ou prolyl hydroxylase, un agent stimulant la synthèse de macromolécules dermiques ou épidermiques et/ou empêchant leur dégradation, un agent stimulant la prolifération des fibroblastes et des kératinocytes et/ou la différenciation des kératinocytes, un agent myorelaxant, un composé diminuant l'irritation d'origine neurogène, un agent anti-microbien, un agent tenseur, un agent anti-pollution ou anti-radicalaire, un agent apaisant capable d'inhiber au moins une enzyme choisie parmi les phospholipases A2, les lipoxygénases et/ou les prostaglandines humaines synthétases.

La présente invention concerne également une composition cosmétique renfermant, dans un milieu physiologiquement acceptable, au moins un dérivé de la DHEA de formule (I) tel que défini précédemment et au moins un filtre UV, choisi parmi les filtres UVA et/ou UVB et/ou au moins un pigment inorganique éventuellement enrobé.

Par "milieu physiologiquement acceptable", on entend un milieu compatible avec la peau, et/ou ses phanères.

Pour donner un ordre de grandeur, la composition selon l'invention peut renfermer de 0,00001% à 10% en poids de dérivé de la DHEA de formule (I) tel que défini précédemment, par rapport au poids total de la composition. De préférence toutefois, cette composition renfermera de 0,001% à 5% en poids de dérivé de la DHEA de formule (I) tel que défini précédemment, par rapport au poids total de la composition.

On détaillera maintenant les différents composés susceptibles d'être introduits dans la composition selon l'invention.

### 1. Agents desquamants et hydratants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de *Saphora japonica* ; les hydroxystilbènes dont en particulier le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux: l'EDTA ; l'acide N-acyl-N,N',N' éthylène diaminetriacétique ; les composés aminosulfoniques et en particulier l'acide (N-(2 hydroxyéthyl) piperazine-N'-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949) ; le miel ; les dérivés de sucre tels que l'O-octanoyl-6-D-maltose et la N-acétyl glucosamine.

Par "agent hydratant", on entend :
- soit un composé agissant sur la fonction barrière, en vue de maintenir l'hydratation du stratum corneum, ou un composé occlusif. On peut citer les céramides, les composés à base sphingoides, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques, la vaseline, et la lanoline;
- soit un composé augmentant directement la teneur en eau du stratum corneum, tel que le thréalose et ses dérivés, l'acide hyaluronique et ses dérivés, le glycérol, le pentanediol, les pidolates, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine.
- Soit un composé activant les glandes sébacées tels que les dérivés stéroidiens (dont la DHEA) et la vitamine D et ses dérivés.

Ces composés peuvent représenter de 0,001% à 30%, et de préférence de 0,01 à 20%, du poids total de la composition selon l'invention.

La composition selon la présente invention comprenant les agents desquamants et hydratants cités ci-dessus est avantageusement destinée à la prévention ou au traitement du dessèchement de la peau et notamment des xéroses.

### 2. Agent dépigmentant ou propigmentant

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Comme agent pro-pigmentant, on peut citer l'extrait de pimprenelle (*Sanguisorba officinalis*) commercialisé par la société MARUZEN et les extraits de chrysanthème (*Chrysanthemum morifolium*).

La composition selon la présente invention comprenant les agents dépigmentants cités ci-dessus est avantageusement destinée à la prévention ou au traitement des hyperpigmentations, en particulier des taches pigmentaires liées au vieillissement de la peau.

De son côté, la composition renfermant les agents pro-pigmentants cités précédemment est de préférence destinée au traitement de la canitie.

### 3. Agent anti-glycation

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (*Vaccinium angusfifolium*) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 99/16166, FR 00/08158, FR 99/09267 et FR 99/16168, respectivement. Le resvératrol est particulièrement préféré pour une utilisation dans cette invention.

La composition selon l'invention comprenant un agent anti-glycation tel que défini ci-dessus peut avantageusement être utilisée pour prévenir ou traiter les signes du vieillissement cutané, en particulier pour prévenir ou traiter la perte de tonicité et/ou d'élasticité de la peau.

### 4. Inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

La composition selon l'invention comprenant un inhibiteur de NO-synthase tel que défini ci-dessus peut avantageusement être utilisée pour prévenir ou traiter les signes du vieillissement cutané et/ou les peaux sensibles.

### 5. Inhibiteur de 5α-réductase

Lorsque la composition selon l'invention comprend un inhibiteur de 5α-réductase, celui-ci peut notamment être choisi parmi :
- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de *Cinnamomum zeylanicum* commercialisé notamment par la société SEPPIC sous la dénomination commerciale Sepicontrol A5® ;
- un extrait de *Laminaria saccharina* commercialisé notamment par la société SECMA sous la dénomination commerciale Phlorogine® ;
- un extrait de *Spiraea ulmaria* commercialisé notamment par la société SILAB sous la dénomination commerciale Sebonormine® ;
- des extraits de végétaux des espèces *Arnica montana, Cinchona succirubra, Eugenia caryophyllata, Humulus lupulus, Hypericum perforatum, Mentha* piperita, *Rosmarinus officinalis, Salvia oficinalis* et *Thymus vulgaris,* tous commercialisés par exemple par la société MARUZEN ;
- un extrait de *Serenoa repens* commercialisé notamment par la société EUROMED;
- des extraits de plantes du genre Silybum ;
- des extraits végétaux contenant des sapogénines et en particulier les extraits de Dioscorées riches en diosgénine ou hécogénine ; et
- des extraits d'*Eugenia caryophyllata* contenant de l'eugenol ou du glucoside d'eugenyle.

L'inhibiteur de 5α-réductase représente par exemple de 0,001% à 10%, et de préférence de 0,01 à 5%, du poids total de la composition selon l'invention. Lorsque celle-ci renferme un tel composé, elle est particulièrement bien adaptée à prévenir ou traiter la séborrhée et/ou l'hirsutisme et/ou l'alopécie androgéno-dépendante.

### 6. Inhibiteur de lysyl et/ou prolyl hydroxylase

Des exemples préférés d'inhibiteurs de lysyl et/ou prolyl hydroxylase utilisables dans la composition selon la présente invention sont le 2,4-diamino-pyrimidine 3-oxyde ou 2,4-DPO décrit dans la demande de brevet WO 96/09048 et le 2,4-diamino-6-pipéridino pyrimidine 3-oxyde ou "Minoxidil" décrit dans les brevets US-4,139,619 et US-4,596,812.

Ces composés sont par exemple présents dans la composition selon l'invention à hauteur de 0,001 à 5% en poids et, mieux, à hauteur de 0,01 à 5% en poids, par rapport au poids total de la composition.

La composition renfermant l'inhibiteur de lysyl et/ou prolyl hydroxylase et le dérivé de la DHEA de formule (I) selon l'invention est avantageusement utilisée pour le traitement de l'alopécie.

### 7. Agent stimulant la synthèse de macromolécules dermiques ou épidermiques et/ou empêchant leur dégradation

Parmi les actifs stimulant les macromolécules du derme, on peut citer ceux qui agissent :
- soit sur la synthèse du collagène, tels que les extraits de *Centella asiatica ;* les asiaticosides et dérivés ; l'acide ascorbique ou vitamine C et ses dérivés ; les peptides de synthèse tels que la iamin, le biopeptide CL ou le palmitoyloligopeptide commercialisé par la société SEDERMA ; les peptides extraits de végétaux, tels que l'hydrolysat de soja commercialisé par la société COLETICA sous la dénomination commerciale Phytokine® ; les hormones végétales telles que les auxines et l'acide cinnamique et ses dérivés tels que décrits dans la demande de brevet européen publiée sous le numéro 0 925 779 ;
- soit sur la synthèse d'élastine, tels que l'extrait de *Saccharomyces cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie® ;
- soit sur la synthèse des glycosaminoglycanes, tels que le produit de fermentation du lait par *Lactobacillus vulgaris,* commercialisé par la société BROOKS sous la dénomination commerciale Biomin yogourth® ; l'extrait d'algue brune *Padina pavonica* commercialisé par la société ALBAN MÜLLER sous la dénomination commerciale HSP3® ; et l'extrait de *Saccharomyces cerevisiae* disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9. On peut citer: les rétinoïdes et dérivés, les isoflavonoïdes, les oligopeptides et les lipopeptides, les lipoaminoacides, l'extrait de malt commercialisé par la société COLETICA sous la dénomination commerciale Collalift® ; les extraits de myrtille ou de romarin ; les caroténoïdes dont en particulier le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon, de sauge ou d'extraits de sauge (tels que décrits dans la demande de brevet français numéro 00 10203) ;
- soit sur l'inhibition des sérine protéases telles que l'élastase leucocytaire ou la cathepsine G. On peut citer : l'extrait peptidique de graines de *légumineuses (Pisum sativum*) commercialisé par la société LSN sous la dénomination commerciale Parelastyl® ; et les héparinoïdes, les pseudodipeptides.

Parmi les actifs stimulant les macromolécules épidermiques, telles que la filaggrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® ; et l'extrait de zooplancton Salina commercialisé par la société SEPORGA sous la dénomination commerciale GP4G®.

La composition selon l'invention renfermant un ou plusieurs des composés ci-dessus convient particulièrement bien à une utilisation dans la prévention ou le traitement des signes cutanés du vieillissement, en particulier de la perte de fermeté et/ou d'élasticité de la peau.

### 8. Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les gibberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; les extraits de tourteaux de noix commercialisés par la société GATTEFOSSE ; et les extraits de *Solanum tuberosum* commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl®.

La composition selon l'invention comprenant ces composés est préférentiellement destinée à être utilisée pour prévenir ou traiter les signes cutanés du vieillissement.

### 9. Agent myorelaxant

Les agents myorelaxants utilisables dans la composition selon l'invention comprennent les inhibiteurs calciques tels que l'alvérine et ses sels, les ouvreurs de canaux chlore tel que le Diazepam, et les inhibiteurs de catécholamines et d'acétylcholine tels que l'hexapeptide argireline R commercialisé par la société ILIPOTEC.

La composition selon l'invention comprenant ces composés est préférentiellement destinée à être utilisée pour prévenir ou traiter les signes cutanés du vieillissement et en particulier les rides.

### 10. Agent anti-microbien

Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorocarbanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, , le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le farnesol, les phytosphingosines et leurs mélanges.

Les agents antimicrobiens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.

A titre d'exemple, l'agent antimicrobien peut être utilisé dans la composition selon l'invention en une quantité représentant de 0,1 à 20%, et de préférence de 0,1 à 10%, du poids total de la composition.

La composition renfermant le dérivé de la DHEA de formule (I) et l'agent anti-microbien convient particulièrement bien à une utilisation dans le traitement des peaux grasses à tendance acnéique, l'acné, ou les pellicules du cuir chevelu.

### 11. Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21.
(2) les protéines végétales de soja ou de blé, et/ou
(3) les silicates de sodium et magnésium (Laponites).

Les compositions selon l'invention comprenant les agents tenseurs ci-dessus sont avantageusement destinées au traitement des signes cutanés du vieillissement, en particulier des rides et ridules.

### 12. Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chlorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou *Eichhornia crassipes* ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'acide éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (*Eichhornia crassipes*) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le coenzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

Les compositions selon l'invention comprenant les agents anti-pollution et/ou anti-radicalaires ci-dessus sont avantageusement destinées à la prévention ou au traitement des signes cutanés du vieillissement, en particulier des rides et de la perte de fermeté et d'élasticité de la peau et de la deshydratation. Elles sont en variante destinées à la prévention ou au traitement du teint terne.

### 13. Filtre UVA et/ou UVB et pigments inorganiques éventuellement enrobés

La composition selon l'invention peut renfermer un ou plusieurs filtres UV capables de filtrer le rayonnement UVA et/ou UVB.

Comme composés capables de filtrer le rayonnement UVA, on peut notamment citer :
(1) les dérivés de benzophénone, par exemple :
   - la 2,4-dihydroxybenzophénone (benzophénone-1) ;
   - la 2,2',4,4'-tétra-hydroxybenzophénone (benzophénone-2) ;
   - la 2-hydroxy-4-méthoxy-benzophénone (benzophénone-3), disponible auprès de la société BASF sous la dénomination commerciale UVINUL M40 ;
   - l'acide 2-hydroxy-4-méthoxy-benzophénone-5-sulfonique (benzophénone-4) ainsi que sa forme sulfonate (benzophénone-5), disponible auprès de la société BASF sous la dénomination commerciale UVINUL MS40 ;
   - la 2,2'-dihydroxy-4,4'-diméthoxy-benzophénone (benzophénone-6) ;
   - la 5-chloro-2-hydroxybenzophénone (benzophénone-7) ;
   - la 2,2'-dihydroxy-4-méthoxy-benzophénone (benzophénone-8) ;
   - le sel disodique du diacide 2,2'-dihydroxy-4,4'-diméthoxy-benzophénone-5,5'-disulfonique (benzophénone-9) ;
   - la 2-hydroxy-4-méthoxy-4'-méthyl-benzophénone (benzophénone-10) ;
   - la benzophénone-11 ;
   - la 2-hydroxy-4-(octyloxy)benzophénone (benzophénone-12),
   - les benzophénones 3 et 5 étant préférées ;
(2) les dérivés de triazine, et en particulier la 2,4-bis [4-(2-éthyl-hexyloxy)-2-hydroxyphényl-6-(4-méthoxy-phényl)-1,3,5-triazine disponible auprès de la société CIBA GEIGY sous la dénomination commerciale TINOSORB S et le 2,2'-méthylènebis-[6-(2H benzotriazol-2-yl)4-(1,1,3,3-tétraméthylbutyl)phénol] disponible auprès de la société CIBA GEIGY sous la dénomination commerciale TINOSORB M ;
(3) l'acide benzène 1,4 di(3-méthylidènecampho 10-sulfonique), éventuellement sous forme partiellement ou totalement neutralisée, et
(4) leurs mélanges.

Comme composé capable de filtrer le rayonnement UVB, on peut notamment citer :
(1) les dérivés de l'acide salicylique, en particulier le salicylate d'homomenthyle et le salicylate d'octyle ;
(2) les dérivés de l'acide cinnamique, en particulier le p-méthoxycinnamate de 2-éthylhexyle, disponible auprès de la société GIVAUDAN sous la dénomination commerciale Parsol MCX ;
(3) les dérivés de β,β'-diphénylacrylate liquides, en particulier l'α-cyano-α,β' diphénylacrylate de 2-éthylhexyle, ou octocrylène, disponible auprès de la société BASF sous la dénomination commerciale UVINUL N539 ;
(4) les dérivés de l'acide p-aminobenzoïque ;
(5) le 4-méthyl benzylidène camphre disponible auprès de la société MERCK, sous la dénomination commerciale EUSOLEX 6300 ;
(6) l'acide 2-phénylbenzimidazole 5-sulfonique vendu sous la dénomination commerciale « EUSOLEX 232 » par la société MERCK
(7) les dérivés de 1,3,5-triazine, en particulier :
   - la 2,4,6-tris[p-(2'-éthylhexyl-1'-oxycarbonyl)anilino]-1,3,5-triazine, disponible auprès de la société BASF sous la dénomination commerciale UVINUL T150, et
   - le composé répondant à la formule (A) suivante : 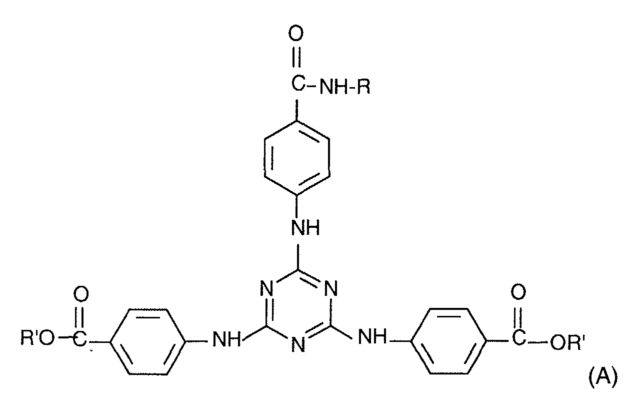
   dans laquelle R' désigne un radical éthyl-2 hexyle et R désigne un radical tert- butyle, disponible auprès de la société SIGMA 3V sous la dénomination commerciale UVASORB HEB ;
(8) leurs mélanges.

Comme composé capable de filtrer le rayonnement UVA et UVB, on peut en particulier citer :
(1) des extraits végétaux, en particulier de Romarin (acide rosmarinique) et du genre Leontopodium, en particulier une espèce végétale choisie parmi *Leontopodium alpinum* ou de *Leontopodium stracheyi* ;
(2) la silicone benzotriazole répondant à la formule générale (B) suivante :

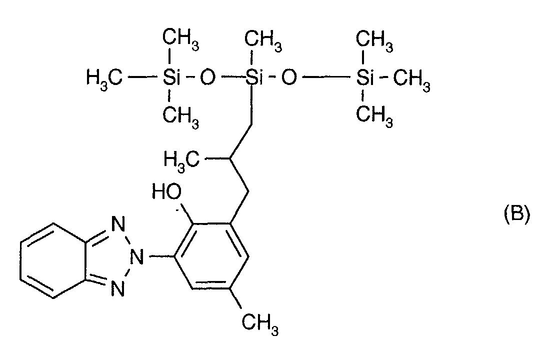

Cette silicone benzotriazole, ainsi que son mode de préparation, sont décrits notamment dans la demande FR-A-2 642 968.

Comme pigments inorganiques éventuellement enrobés, on peut citer les nanopigments d'oxyde de titane, de fer, de zinc, de zirconium ou de cérium éventuellement enrobés d'alumine et/ou de stéarate d'aluminium.

### 14. Composés diminuant l'irritation d'origine neurogène

Comme composés diminuant l'irritation d'origine neurogène on peut citer :
- les antagonistes de substance P et en particulier ceux cités dans le brevet EP 0 680 749, les extraits d'au moins une bactérie filamenteuse non photosynthétique, particulièrement des souches de *Vitreoscilla filiformis* décrits dans le brevet EP 0 761 204, les eaux thermales décrites dans le brevet EP 0 764 440, les extraits d'au moins un végétal de la famille des Rosacées, particulièrement de l'espèce *Rosa gallica* décrits dans la demande brevet européen publiée sous le numéro n° 0 906 752 et les sels de métaux alcalinoterreux décrits dans les demandes de brevets européens publiées sous les numéros 0 737 471 et 0 770 392 ;
- les antagonistes de CGRP, en particulier ceux cités dans le brevet EP 0 765 668 et notamment les extraits d'Iridacées, particulièrement de l'espèce *Iris pallida* ;
- les inhibiteurs de NO-synthase ;
- les antagonistes de bradykinine et en particulier ceux cités dans la demande de brevet européen publiée sous le numéro 0 909 556;
- les antagonistes de cytokines ;
- les antagonistes d'histamine ;
- les antagonistes de l'Interleukine 1 et/ ou du facteur de nécrose tumorale de type α (TNFα) et en particulier ceux cités dans les demandes de brevets européens publiées sous les numéros 0 892 642 et 0 764 444, particulièrement le peptide Modulène, le tripéptide Lysine-Proline-Valine (KPV) et un extrait d'au moins un végétal de la famille des Labiées, particulièrement de l'espèce *Rosmarinus officinalis.*
- les agents bloqueurs de canaux sodiques choisi notamment parmi : l'Amiloride, la Quinidine, la Quinidine sulfate, l'Apamine, la Cyproheptadine, la Loperamide et la N-acétylprocaïnamide
- les agents ouvreurs de canaux potassiques et notamment le Minoxidil et ses dérivés,

### 15. agents apaisants capables d'inhiber au moins une enzyme choisie parmi les phospholipases A2, les lipoxygénases et/ou les prostaglandines humaines synthétases.

- les triterpènes pentacycliques et les extraits de plante (notamment *Glycyrrhiza glabra*) en contenant, en particulier l'acide oléanolique et ses sels, l'acide β glycyrrhétinique et ses sels et/ou ses dérivés tels que l'acide glycyrrhétique monoglucuronide, le glycyrrhétinate de stéaryle, l'acide 3-stéaroyloxy glycyrrhétique ;
- l'acide bétulinique et ses sels ;
- les extraits de *Paeonia suffruticosa* ;
- les extraits de *Paeonia lactiflora* ;
- l'huile de calophyllum ;
- les phycosaccharides, en particulier hydrolysed algin® ou hydrolysed algin and zinc sulfate® de la société Codif ;
- l'huile de Canola ;
- l'huile de Tamanu ;
- l'alphabisabolol ;
- les extraits de camomille ;
- l'allantoine ;
- Le diesterphosphorique de vitamine E et C, en particulier le Sépivital EPC® de Seppic ;
- les huiles insaturées en oméga 3 telles que les huiles de rosier musca, les huiles de cassis, des huiles d'echium, les huiles de poisson ;
- Les extraits de plancton, en particulier l'Oméga plancton® de Secma ;
- L'associatition de sodium palmitoylproline et de *Nymphéa alba,* en particulier le Seppicalm VG® de Séppic ;
- les extraits du pygeum ;
- Les extraits de *Boswellia serrata*, en particulier le Soothex® de Quest ;
- Les extraits de *Centipeda cunnighami*, en particulier le phytoplenolin® de Bio-Botanica ;
- Les extraits *d'Hélianthus annuus*, en particulier l'Hélioxine® de Silab ;
- Les extraits de *Linum usitatissimum*, en particulier la Sensiline® de Silab ;
- les tocotrienols ;
- les extraits de *Cola nitida* ;
- le piperonal ;
- les extraits de clou de girofle ;
- les extraits d'épilobe (*Epilobium angustifolium*) ;
- les extraits d'*Aloe vera* ;
- la cortisone ;
- l'hydrocortisone ;
- l'indométacine ;
- la bétaméthasone.

Outre le ou les composés décrits ci-dessus, la composition selon l'invention renferme généralement une quantité efficace de dérivés de la DHEA de formule (I) tels que définis précédemment, suffisante pour obtenir l'effet recherché. Elle contient ainsi, par exemple, de 0,00001% à 10% en poids dudit dérivé de la DHEA de formule (I), par rapport au poids total de la composition, et, mieux, de 0,001% à 5% en poids dudit dérivé de la DHEA de formule (I), par rapport au poids total de la composition.

Les compositions selon l'invention peuvent être destinées à une application cosmétique ou pharmaceutique, particulièrement dermatologique. De préférence les compositions selon l'invention sont destinées à une application cosmétique.

Les compositions selon l'invention peuvent être utilisées à des fins cosmétiques, pour améliorer l'apparence des matières kératiniques, en particulier pour prévenir ou traiter les signes cutanés du vieillissement et/ou le teint terne et/ou les troubles de pigmentation de la peau ou des cheveux et/ou le dessèchement de la peau et/ou l'hyperséborrhée et/ou les imperfections liées à l'hyperséborrhée et/ou les peaux sensibles et/ou les pellicules et/ou la chute des cheveux et/ou la canitie.

Les compositions selon l'invention sont de préférence adaptées à une application topique sur les matières kératiniques telles que la peau, les cheveux, les cils ou les ongles. Elles peuvent se présenter sous toutes les formes galéniques normalement utilisées pour ce type d'application, notamment sous forme d'une solution aqueuse ou huileuse, d'une émulsion huile-dans-eau ou eau-dans-huile, d'une émulsion siliconée, d'une microémulsion ou nanoémulsion, d'un gel aqueux ou huileux ou d'un produit anhydre liquide, pâteux ou solide.

Ces compositions peuvent être plus ou moins fluide et avoir l'aspect d'une crème blanche ou colorée, d'une pommade, d'un lait, d'une lotion, d'un sérum, d'une pâte, d'une mousse ou d'un gel. Elles peuvent éventuellement être appliquées sur la peau sous forme d'aérosol, de patch ou de poudre. Elles peuvent également se présenter sous forme solide, et par exemple sous forme de stick. Elles peuvent être utilisées comme produit de soin et/ou comme produit de maquillage de la peau. Elles peuvent en variante se présenter sous forme de shampooing ou d'après-shampooing.

De façon connue, la composition de l'invention peut contenir également les adjuvants habituels dans le domaine cosmétique, tels que les gélifiants hydrophiles ou lipophiles, les conservateurs, les antioxydants, les solvants, les parfums, les charges, les pigments, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses du dérivé de la DHEA de formule (I) selon l'invention.

Lorsque la composition selon l'invention est une émulsion, la proportion de la phase grasse peut aller de 2 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le co-émulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

La présente invention se rapporte encore à un procédé de traitement cosmétique des matières kératiniques comprenant l'application topique sur celles-ci d'une composition renfermant, dans un milieu physiologiquement acceptable, au moins un dérivé de la DHEA de formule (I) tel que défini précédemment, seul ou en association avec au moins un composé tel que décrit précédemment.

Elle se rapporte plus particulièrement à un procédé de traitement cosmétique des signes cutanés du vieillissement et/ou du teint terne et/ou des troubles de pigmentation de la peau ou des cheveux et/ou du dessèchement de la peau et/ou de l'hyperséborrhée et/ou des imperfections liées à l'hyperséborrhée et/ou des peaux sensibles et/ou des pellicules et/ou de la chute des cheveux et/ou de la canitie, comprenant l'application topique sur la peau ou les cheveux d'une composition renfermant, dans un milieu physiologiquement acceptable, au moins un dérivé de la DHEA de formule (I) tel que défini précédemment seul ou en association avec au moins un composé tel que décrit précédemment.

L'invention sera maintenant illustrée par les exemples non limitatifs suivants. Dans les exemples de composition, les quantités sont indiquées en pourcentage pondéral.

### EXEMPLES

### Exemple 1 : Crème hydratante

| Phase A | |
|---|---|
| Copolymère acrylate / C₁₀-₃₀ acrylate | 0,5 % |
| Eau | 12,0 % |

| Phase B | |
|---|---|
| Polyisobutène hydrogéné | 5,0 % |
| Androst-5-en-17-one, 3-(acetyloxy)-7-methoxy-, (3β,7α) | 0,1 % |
| Cyclohexasiloxane | 6,0 % |

| Phase C | |
|---|---|
| Triéthanolamine | 1,0 % |
| Glycérine | 6,0 % |
| EDTA | 0,2 % |
| Conservateurs | 0,5 % |
| Glycine | 2,0 % |
| Polyacrylamide et C₁₃-₁₄ isoparaffine et laureth-7 | 1,0 % |
| Eau qsp | 100 % |

Cette composition peut être préparée de la manière suivante. Le polymère de la phase A est dispersé dans l'eau à 40°C. Les constituants de la phase B sont chauffés à 70°C jusqu'à dissolution complète, puis la température est ramenée à 40°C. Les constituants de la phase C sont mélangés à 50°C. La phase B est ensuite introduite dans la phase A à 40°C sous agitation, puis la phase C leur est ajoutée.

La composition ci-dessus permet de réhydrater et lisser les peaux sèches.

### Exemple 2 : Crème hydratante

On prépare la composition suivante de manière classique pour l'homme du métier.

| *Phase A* | |
|---|---|
| Eau déminéralisée QSP | 100 % |
| Conservateurs | 0,5 % |
| Carbomer | 0,4 % |
| Glycérine | 7,0 % |
| | |

| *Phase B1* | |
|---|---|
| Stéarate de sorbitane oxyéthyléné (200 OE) | 0,9 % |
| | |

| *Phase B2* | |
|---|---|
| Stéarate de PEG-100 et stéarate de glycéryle | 2,1 % |
| Isononanoate d'isononyle | 10,0 % |
| Vaseline | 2,0 % |
| Octyldodécanol | 10,0 % |
| Androst-5-en-17-one, 3-(acetyloxy)-7-methoxy-, (3β,7α) | 0,5 % |
| Butylhydroxytoluène | 0,1 % |
| Filtre UV | 1,0 % |
| Céramides | 0,5 % |
| | |

| *Phase C* | |
|---|---|
| Eau | 2,0 % |
| Triethanolamine | 0,5 % |
| Urée | 1,0 % |

Cette crème peut être utilisée dans le soin des peaux sèches.

### Exemple 3 : Crème anti-âge

| | |
|---|---|
| Copolymère acrylate / C₁₀-₃₀ acrylate | 0,5 % |
| Eau | 12,0 % |
| Isononanoate d'isononyle | 5,0 % |
| Androst-5-ene-7,17-dione, 3-(acetyloxy)-17 oxime (3β) | 0,1 % |
| Cyclohexasiloxane | 5,0 % |
| Octyl méthoxycinnamate | 1,0 % |
| Triéthanolamine | 1,0 % |
| Glycérine | 6,0 % |
| Conservateurs | 0,5 % |
| Polyacrylamide et C₁₃-₁₄ isoparaffine et laureth-7 | 1,0 % |
| Eau qsp | 100 % |

Cette composition peut être préparée de la manière suivante. Le polymère de la phase A est dispersé dans l'eau à 40°C. Les constituants de la phase B sont chauffés à 70°C jusqu'à dissolution complète, puis la température est ramenée à 40°C. Les constituants de la phase C sont mélangés à 50°C. La phase B est ensuite introduite dans la phase A à 40°C sous agitation, puis la phase C leur est ajoutée.

Cette crème peut être utilisée en applications mono- ou bi-quotidiennes pour traiter les signes cutanés du vieillissement, en particulier estomper les rides et ridules.

### Exemple 4: Crème anti-âge

On prépare la composition suivante de manière classique pour l'homme du métier.

| *Phase A* | |
|---|---|
| Eau déminéralisée QSP | 100,0 % |
| Conservateurs | 0,5 % |
| Carbomer | 0,4 % |
| Glycérine | 7,0 % |

| *Phase B1* | |
|---|---|
| Stéarate de sorbitane oxyéthyléné (200 OE) | 0,9 % |
| | |

| *Phase B2* | |
|---|---|
| Stéarate de PEG-100 et stéarate de glycéryle | 2,1 % |
| Isononanoate d'isononyle | 10,0 % |
| Octyldodécanol | 10,0 % |
| Androst-5-ene-7,17-dione, 3-(acetyloxy)-17 oxime (3β) | 0,1 % |
| Butylhydroxytoluène | 0,1 % |
| Filtre UV | 1,0 % |
| | |

| *Phase C* | |
|---|---|
| Eau | 2,0 % |
| Triethanolamine | 0,5 % |
| Extrait de Centella Asiatica | 1,0 % |
| Palmitoyl pentapeptide (Matrixyl® de SEDERMA) | 0,1 % |

Cette crème est utile comme crème de jour raffermissante.

### Exemple 5 : Gel de nettoyage pour peaux grasses

On prépare de manière classique pour l'homme du métier la composition suivante :

| | |
|---|---|
| Lauryl phosphate | 6,50% |
| Décyl glucoside | 16,25 % |
| Polyquaternium-7 | 5,70 % |
| Tétrastéarate de pentaérythrityle oxyéthyléné (150 OE) | 0,50% |
| Glycérine | 3,50% |
| Sorbitol | 3,50% |
| Hydroxyde de potassium | 1,70% |
| Hydroxypropylcellulose | 0,20% |
| EDTA disodique | 0,05% |
| Chlorure de sodium | 0,10% |
| Androst-5-en-17-one, 3-(acetyloxy)-7-fluoro-, (3β,7β) | 0,5 % |
| Conservateurs | 0,30% |
| Eau QSP | 100 % |

Ce gel permet de réguler les sécrétions de sébum et d'atténuer les imperfections cutanées.

### Exemple 6 : Patch anti-taches

On prépare un patch comprenant la composition suivante :

| | |
|---|---|
| Eau | 40,0 % |
| Alcool QSP | 100 % |
| Glycérine | 7,0 % |
| Androst-5-en-17-one, 3-(acetyloxy)-7-fluoro-, (3β,7β) | 0,1 % |
| Alcool polyvinylique | 5,0 % |
| Acide kojique | 0,5 % |

Ce patch peut être appliqué sur les mains et le décolleté pour estomper les taches pigmentaires, en particulier les taches de sénescence.

### Exemple 7 : Lotion capillaire anti-chute

On prépare de manière classique pour l'homme du métier la composition suivante :

| | |
|---|---|
| Eau | 25,0 % |
| Glycérine | 7,0 % |
| Androst-5-ene-7,17-dione, 3-(acetyloxy)-17 oxime (3β) | 0,5 % |
| Alcool QSP | 100 % |

Cette lotion est efficace pour prévenir la chute des cheveux.

## Revendications

1. Utilisation cosmétique pour améliorer l'apparence des matières kératiniques d'au moins un dérivé de la DHEA de formule (I) suivante : 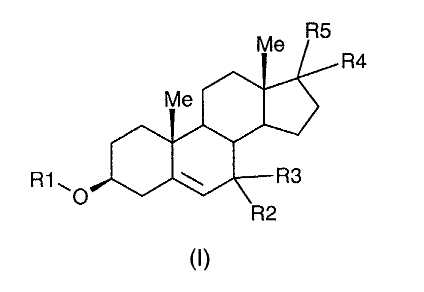 dans laquelle :
R1 est choisi parmi :
- un atome d'hydrogène ;
- un groupe alkyle en C1-C12, saturé ou insaturé, linéaire ou ramifié, cyclique pouvant éventuellement contenir un ou plusieurs hétéroatomes, et éventuellement substitué par un ou plusieurs groupes choisis parmi -OR' et/ou -SR' et/ou -COOR' et/ou -NR'R' et/ou halogène et/ou perfluoroalkyle et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
- un groupe alkylcarbonyle, dont la partie alkyle en C1-C24 est saturée ou insaturée, linéaire ou ramifiée, cyclique, et éventuellement substituée par un ou plusieurs groupes choisi parmi -OR' et/ou -SR' et/ou -COOR' et/ou -NR'R' et/ou halogène et/ou perfluoroalkyle et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
- un groupe phényle, éventuellement fonctionnalisé par un ou plusieurs groupes -OR' et/ou -SR' et/ou -COOR' et/ou -NR'R' et/ou halogène et/ou perfluoroalkyle et/ou sulfate et/ou phosphate et/ou aryle et/ou hétérocycle ;
- un groupe benzyle, éventuellement fonctionnalisé par un ou plusieurs groupes -OR' et/ou -SR' et/ou -COOR' et/ou -NR'R' et/ou halogène et/ou perfluoroalkyle et/ou sulfate et/ou phosphate et/ou aryle et/ou hétérocycle ;
- un groupe arylcarbonyle, de préférence un phénylcarbonyle, ou un groupe arylalkylcarbonyle, de préférence un benzylcarbonyle, éventuellement substitué par un ou plusieurs groupes -OR' et/ou -SR' et/ou -COOR' et/ou -NR'R' et/ou halogène et/ou perfluoroalkyle et/ou sulfate et/ou phosphate et/ou aryle et/ou hétérocycle ;
- un groupe O=P(OH)OR' ;
- un groupe (O)₂SOR' ;
- un groupe (O)₂SR' ;
- un groupe trialkylsilyle (SiR'₃) dans lequel les 3 groupes R' peuvent être identiques ou différents ;
- un groupe alkyloxycarbonyle (R'OCO) ;
- un groupe alkylaminocarbonyle (R'NR'''CO) ;
- un hydrate de carbone de 3 à 100 atomes de carbones et leurs dérivés ;
R4 et R5 représentent :
- ensemble un groupe choisi parmi :
- un groupe céto (=O) ;
- un groupe =CHR' ;
- un groupe =NR' ;
- un groupe =NCOR' ;
- chacun un groupe -OR" identique, avantageusement les deux groupes -OR" forment ensemble un cycle 1,3-dioxolane ou 1,3-dioxane ;
- chacun un groupe identique ou différent choisi parmi :
- un atome d'hydrogène ;
- un groupe -NR'R' ;
- un groupe -NHCOR' ;
Lorsque R3 représente un atome d'hydrogène, R2 est choisi parmi :
- un groupe -OR6 dans lequel R6 a les mêmes définitions que celles données précédemment pour R1 ;
- un groupe amine -N(R7)₂ dans lequel les substituants R7, identiques ou différents, ont les mêmes définitions que celles données précédemment pour R1;
- un groupe N-sulfonamide (-NR'''SO₂R') ou N-sulfonate (-NR'''SO₃R'), de préférence sous forme de sel de métal alcalin ;
- un groupe urée (-NR'''CONR'R') ;
- un groupe alkylamide (-NR'''COR') ;
- un groupe N-carbamate (-NR'''COOR') ;
- un groupe thiol (-SR') ;
- un groupe thiophényl (-SPh) ;
- un groupe sulfone (-SO₂R') ;
- un groupe sulfoxide (-SOR') ;
- un halogène ;
- un groupe alkylsilane (-SiR'₃) dans lequel les 3 groupes R' peuvent être identiques ou différents ;
R2 et R3 peuvent également représenter ensemble un groupe choisi parmi :
- un méthylène (=CHR') ;
- une imine (=NHR') ;
- une oxime (=NOR') ;
- une hydrazone (=N-NR'R')
- un groupe céto (=O) ;
R2 et R3 peuvent encore représenter chacun un groupe -OR" dans lequel chacun des R" représente des chaînes alkyles en C1-C6, de préférence en C1-C3 et formant ensemble avantageusement un cycle de préférence à 5 ou 6 chaînons ;
R' est choisi parmi un atome d'hydrogène, un groupe alkyle en C1-C12, de préférence en C1-C6, saturé ou insaturé, linéaire ou ramifié, cyclique pouvant éventuellement contenir un ou plusieurs hétéroatomes, éventuellement fonctionnalisé par un ou plusieurs groupes -OR"', -COOR"', halogène, -NR'''R'''; ou par un groupe aryle, de préférence un phényle, éventuellement fonctionnalisé par un ou plusieurs groupes -OR''', -COOR''', halogène ou -NR'''R''' ;
R''' représente un atome d'hydrogène, une chaîne alkyle, de préférence en C1-C6, saturée ou insaturée, linéaires ou ramifiées ou cycliques,
étant entendu que dans chacun des groupes -NR'R' et -NR'''R''', les substituants R', respectivement R''', sont identiques ou différents ;
à l'exception des dérivés de la DHEA de formule (I) pour lesquels :
- R2 et R3 représentent ensemble un groupe céto ;
- R4 et R5 représentent ensemble un groupe céto, et
- R1 est tel que défini précédemment ;
à l'exception des dérivés de la DHEA de formule (A) 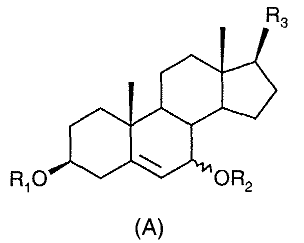 dans laquelle :
- R₁ est choisi parmi : un atome d'hydrogène, les fonctions ester d'acide organique de 1 à 24 atomes de carbone, ester sulfurique ou ester phosphorique, ou ether carboné de 1 à 24 atomes de carbone comprenant zéro ou plusieurs atomes d'azote, les ethers d'hydrates de carbones de 3 à 100 atomes de carbone et leurs dérivés comprenant ou non un ou plusieurs atomes d'azote ;
- R2 est choisi parmi : un atome d'hydrogène ou une fonction ester d'acide gras de 1 à 24 atomes de carbone ;
- R3 est choisi parmi : un atome d'hydrogène, un groupe -OH, les groupes de formules : -CO-R₄, -CHOH-R₄, =CH-CH₃, =COH-CH₃, -CHR₄-CH₃, =O, dans lesquels R₄ est un groupe alcoyle comprenant de 1 à 10 atomes de carbone substitué ou non.

2. Utilisation selon la revendication 1, **caractérisée en ce que** R1 représente un groupe alkyle en C1-C6, saturé ou insaturé, linéaire ou ramifié, ou cyclique pouvant contenir un ou plusieurs hétéroatomes, et éventuellement substitué par un ou plusieurs groupes choisis parmi -OR' et/ou -SR' et/ou -COOR' et/ou -NR'R' et/ou halogène.

3. Utilisation selon la revendication 1, **caractérisée en ce que** R1 représente un groupe alkylcarbonyle, dont la partie alkyle en C1-C20, est saturée ou insaturée, linéaire ou ramifié ou cyclique et éventuellement substituée par un ou plusieurs groupes choisis parmi -OR' et/ou -SR' et/ou -COOR' et/ou -NR'R' et/ou halogène.

4. Utilisation selon la revendication 3, **caractérisée en ce que** R1 représente un groupe alkylcarbonyle, avec une partie alkyle en C6-C18.

5. Utilisation selon la revendication 1, **caractérisée en ce que** R3 représente un atome d'hydrogène et R2 est choisi parmi :
- un groupe -OR6 dans lequel R6 a les mêmes définitions que celles données pour R1 dans l'une quelconque des revendications 2 à 4 ;
- un groupe amine -N(R7)₂ dans lequel les substituants R7, identiques ou différents, ont les mêmes définitions celles données pour R1 dans l'une quelconque des revendications 2 à 4 ;
- un groupe thiol (-SR') ;
- un halogène.

6. Utilisation selon l'une quelconque des revendications précédentes, **caractérisée en ce que** chacun des groupes R' et R'" représente un groupe choisi parmi un atome d'hydrogène et un groupe alkyle en C1-C6.

7. Utilisation selon l'une quelconque des revendications précédentes, **caractérisée en ce que** chacun des groupes R' et R'" représente un groupe choisi parmi un atome d'hydrogène, un méthyle, un éthyle, un butyle, un propyle, un isopropyle, un *tert*-butyle.

8. Utilisation selon l'une quelconque des revendications précédentes, **caractérisée en ce que** chacun des groupes -NR'R' et -NR'''R''' représente un acide aminé, de préférence choisi parmi la L-alanine, la L-arginine, la L-asparagine, acide L-aspartique, la L-cystéine, la L-glutamine, acide L-glutamique, la glycine, la L-histidine, la L-isoleucine, la L-leucine, la L-lysine, la L-méthionine, la L-phénylalanine, la L-proline, la L-sérine, la L-thréonine, le L-tryptophane, la L-tyrosine, la L-valine.

9. Utilisation selon l'une quelconque des revendications précédentes, **caractérisée en ce que** le dérivé de la DHEA de formule (I) est choisi parmi au moins un des dérivés suivants :
- Androst-5-en-17-one, 3-(acetyloxy)-7-(benzoyloxy)-, (3β,7α) ;
- Androst-5-en-17-one, 7-hydroxy-3-[[(1-oxopentyl)sulfonyl]oxy]-, (3β,7β) ;
- Androst-5-en-17-one, 3-hydroxy-7-(1-oxo-3-phenylpropoxy)-, (3β,7β) ;
- Androst-5-en-17-one, 7-hydroxy-3-[[3-(4-hydroxy-3-methoxyphenyl)-1-oxo-2-propenyl]oxy]-, (3β) ;
- Androst-5-en-17-one, 7-hydroxy-3-[[(9Z)-1-oxo-9-octadecenyl]oxy]-, (3β) ;
- Pregn-5-en-20-one, 3-(acetyloxy)-7-hydroxy-, (3β,7α) ;
- Butanoic acid, 4-[[[(3β,7Z)-3-hydroxy-17-oxoandrost-5-en-7-ylidene]amino]oxy] ;
- Butanoic acid, 4-[[[(3β,7Z)-3-(acetyloxy)-17-oxoandrost-5-en-7-ylidene]amino]oxy]-, methyl ester ;
- Androst-5-en-17-one, 3-(benzoyloxy)-7-(2-methyl-1-oxopropoxy)-, (3β,7β) ;
- Androst-5-en-17-one, 3-(acetyloxy)-7-fluoro-, (3β,7β) ;
- Androst-5-en-17-one, 3-(acetyloxy)-7-fluoro-, (3β,7α) ;
- Acetic acid, [[[(3β,7Z)-3-hydroxy-20-oxopregn-5-en-7-ylidene]amino]oxy] ;
- Acetic acid, [[[(3β,7Z)-3-(acetyloxy)-20-oxopregn-5-en-7-ylidene]amino]oxy]-, methyl ester ;
- Androst-5-en-17-one, 3-(acetyloxy)-7-bromo-, (3β) ;
- Androst-5-en-17-one, 7-bromo-3-hydroxy-, (3β,7α) ;
- Androst-5-en-17-one, 7-bromo-3-hydroxy-, (3β) ;
- Pregn-5-en-20-one, 7-bromo-3-hydroxy-, acétate ;
- Pregn-5-en-20-one, 3-(acetyloxy)-7-bromo-, (3β)
- Pregn-5-en-20-one, 7α-chloro-3β-hydroxy-, acétate ;
- Pregn-5-en-20-one, 3-(acetyloxy)-7-bromo-, (3β,7β) ;
- Pregn-5-en-20-one, 3-(acetyloxy)-7-bromo-, (3β,7α) ;
- Pregn-5-en-20-one, 7-bromo-3-hydroxy-, benzoate ;
- Pregn-5-en-20-one, 7-bromo-3β-hydroxy-, 4-methylvalerate ;
- Androst-5-en-17-one, 3β,7α-dihydroxy-, disulfate ;
- Acetic acid, [[(3β,7α)-3-hydroxy-17-oxoandrost-5-en-7-yl]thio] ;
- Androst-5-ene-7,17-dione, 3-(acetyloxy)-, 7,17-dioxime, (3β) ;
- Androst-5-en-17-one, 3-(benzoyloxy)-7-bromo-, (3β,7α) ;
- Acetic acid, [[[(3β,7Z)-3-hydroxy-17-oxoandrost-5-en-7-ylidene]amino]oxy] ;
- Acetic acid, [[[(3β,7Z)-3-hydroxy-17-oxoandrost-5-en-7-ylidene]amino]oxy]-, methyl ester ;
- Acetic acid, [[[(3β,7Z)-3-(acetyloxy)-17-oxoandrost-5-en-7-ylidene]amino]oxy]-, methyl ester ;
- Androst-5-en-17-one, 3,7-bis[(trimethylsilyl)oxy]-, (3β,7β) ;
- Androst-5-en-17-one, 7-hydroxy-3-[[(3-methylphenyl)sulfonyl]oxy]-, (3β,7α) ;
- Androst-5-en-17-one, 3-[(3-chlorobenzoyl)oxy]-7-hydroxy-, (3β,7α) ;
- Androst-5-en-17-one, 7-(3-carboxy-1-oxopropoxy)-3-[(1-oxoheptyl)oxy]-, (3β,7α) ;
- Pregn-5-en-20-one, 3,7-bis[(trimethylsilyl)oxy]-, (3β,7α) ;
- Androst-5-en-17-one, 3,7-bis[(trimethylsilyl)oxy]-, (3β,7α) ;
- Acetic acid, [[[(3β)-3-hydroxy-17-oxoandrost-5-en-7-ylidene]amino]oxy] ;
- Androst-5-en-17-one, 3-(acetyloxy)-7-methoxy-, (3β,7α) ;
- Androst-5-en-17-one, 3-(acetyloxy)-7-methoxy-, (3β,7β) ;
- Pregna-5,16-diene-7,20-dione, 3-(acetyloxy)-, 7,20-dioxime, (3β) ;
- Androst-5-en-17-one, 3-(acetyloxy)-7-bromo-, (3β,7α) ;
- Pregn-5-en-20-one, 3β,7α-dihydroxy-, 7-(hydrogen sulfate) ;
- Androst-5-en-17-one, 3-hydroxy-7-(sulfooxy)-, (3β,7α) ;
- Pregn-5-ene-7,20-dione, 3-hydroxy-, bis[(aminoiminomethyl)hydrazone], (3β) ;
- Guanidine, 1,1'-[(3β-hydroxypregn-5-ene-7,20-diylidene)dinitrilo]di-, dihydrochloride ;
- Androst-5-en-17-one, 7-bromo-3β-hydroxy-, benzoate ;
- Androst-5-en-17-one, 3-hydroxy-7-methoxy-, (3β,7α) ;
- Androst-5-en-17-one, 3-hydroxy-7-methoxy-, (3β,7β) ;
- Androst-5-en-7-one, 3-(acetyloxy)-17-(benzoyloxy)-, 7-[O-(phenylmethyl)oxime], (3β,7E,17β) ;
- Androst-5-ene-3,7,17-triol, 17-(hydrogen sulfate), (3β,7β,17β) ;
- Androst-5-en-7-one, 3,17-bis(acetyloxy)-, 7-[O-(3-hydroxypropyl)oxime], (3β,7Z,17β) ;
- Propanoic acid, 3-[[[(3β,7Z,17β)-3,17-bis(acetyloxy)androst-5-en-7-ylidene]amino]oxy]-, methyl ester ;
- Propanoic acid, 3-[[[(3β,7Z,17β)-3,17-bis(acetyloxy)androst-5-en-7-ylidene]amino]oxy] ;
- Androst-5-en-17-one, 7-(acetyloxy)-3-[[(1,1-dimethylethyl)dimethylsilyl]oxy]-, cyclic 17-(1,2-ethanediyl acetal), (3β,7β) ;
- Androst-5-en-7-one, 3-(acetyloxy)-17-[[(1,1-dimethylethyl)dimethylsilyl]oxy]-, cyclic 7-(1,2-ethanediyl acetal), (3β,17β) ;
- Androst-5-ene-7,17-diol, 3-[[(1,1-dimethylethyl)diphenylsilyl]oxy]-, 17-octanoate, (3β,7β,17β) ;
- Butanoic acid, 4-[[[(3β,7Z,17β)-3-(acetyloxy)-17-hydroxyandrost-5-en-7-ylidene]amino]oxy]-, methyl ester ;
- Androst-5-ene-3,7,17-triol, 17-acetate 3-benzoate, (3β,7β,17β) ;
- Acetic acid, [[[(3β,7Z,17β)-3-(acetyloxy)-17-hydroxyandrost-5-en-7-ylidene]amino]oxy]-, methyl ester ;
- Androst-5-ene-3,7,17-triol, 3,17-diacetate 7-methanesulfonate, (3β,17β) ;
- Androst-5-ene-3,17-diol, 7-bromo-, diacetate, (3β,7α,17β) ;
- Androst-5-ene-3,7,17-triol, 3-acetate 17-benzoate, (3β,7α,17β) ;
- Androst-5-ene-3,7,17-triol, 3-acetate 17-benzoate, (3β,7β,17β) ;
- Androst-5-ene-3,17-diol, 7-bromo-, (3β,7α,17β) ;
- Androst-5-ene-3,17-diol, 7-bromo-, (3β,17β) ;
- Androst-5-ene-3,17-diol, 7-bromo-, 3-acetate 17-benzoate, (3β,7α,17β) ;
- Androst-5-ene-3,17-diol, 7-(phenylsulfinyl)-, diacetate, (3β,17β) ;
- Androst-5-ene-3,17-diol, 7-(phenylthio)-, diacetate, (3β,17β) ;
- Androst-5-ene-3,17-diol, 7-bromo-, diacetate, (3β,17β) ;
- Androst-5-ene-3β,17β-diol, 7-bromo-, dibenzoate ;
- Androst-5-ene-3,17-diol, 7-bromo-, 3-acetate 17-benzoate, (3β,7β,17β) ;
- Propanoic acid, 3-[[(3β,7α,17β)-3-(acetyloxy)-17-(benzoyloxy)androst-5-en-7-yl]thio]-, methyl ester ;
- Propanoic acid, 3-[[(3β,7α,17β)-3,17-dihydroxyandrost-5-en-7-yl]thio] ;
- Androst-5-en-7-one, 3-(acetyloxy)-17-(benzoyloxy)-, 7-[O-(phenylmethyl)oxime], (3β,7Z,17β) ;
- Androst-5-ene-3,7,17-triol, 3-acetate 7,17-dibenzoate, (3β,7α,17β) ;
- Androst-5-ene-3,7,17-triol, 3-acetate 7,17-dibenzoate, (3β,7β,17β) ;
- Androst-5-en-17-one, 3-(acetyloxy)-7-bromo-, cyclic 1,2-ethanediyl acetal, (3β,7β) ;
- Androst-5-en-7-one, 3-(acetyloxy)-17-(benzoyloxy)-, 7-[O-(phenylmethyl)oxime], (3β,17β) ;
- Androst-5-en-17-one, 3,7-bis(acetyloxy)-, cyclic 17-(1,2-ethanediyl acetal), (3β,7α) ;
- Androst-5-en-17-one, 3-(acetyloxy)-7-hydroxy-, cyclic 17-(1,2-ethanediyl acetal), (3β,7β) ;
- Androst-5-en-17-one, 3-(acetyloxy)-7-hydroxy-, cyclic 17-(1,2-ethanediyl acetal), (3β,7α) ;
- Androst-5-ene-7,17-dione, 3-(acetyloxy)-, 7,17-dioxime, (3β) ;
- Androst-5-en-3-ol, 7-bromo-, benzoate, (3β) ;
- Androst-5-ene-7,17-dione, 3-(acetyloxy)-, 17-(1,2-ethanediyl acetal), 7-oxime, (3β,7Z) ;
- Acetic acid, [[[(3β,7Z)-3-(acetyloxy)-17,17-[1,2-ethanediylbis(oxy)]androst-5-en-7-ylidene]amino]oxy]-, methyl ester ;
- Androst-5-en-7-one, 3,17-bis[(trimethylsilyl)oxy]-, O-methyloxime, (3β,17β) ;
- Androst-5-en-7-one, 3,17-dihydroxy-, oxime, (3β,17β) ;
- Androst-5-ene-7,17-dione, 3-(acetyloxy)-17 oxime (3β) ;
- Androst-5-ene-7,17-dione -3-(acetyloxy)-7,17dioxime (3β) ;
- Acetic acid, [[[(3β,17β)-3,17-dihydroxyandrost-5-en-7-ylidene]amino]oxy] ;
- Acetic acid, [[[(3β)-17,17-[1,2-ethanediylbis(oxy)]-3-hydroxyandrost-5-en-7-ylidene]amino]oxy] ;
- Silane, [[(3β,7α,17β)-androst-5-ene-3,7,17-triyl]tris(oxy)]tris[trimethyl] ;
- Benzenesulfonic acid, 4-methyl-, [(3β,17β)-3,17-bis(acetyloxy)androst-5-en-7-ylidene]hydrazide ;
- Androst-5-en-7-one, 3,17-bis(acetyloxy)-, oxime, (3β,17β) ;
- Androst-5-en-17-one, 3-(acetyloxy)-7-bromo-, cyclic 1,2-ethanediyl acetal, (3β,7α) ;
- Androst-5-en-17-one, 7-bromo-3-hydroxy-, cyclic 1,2-ethanediyl acetal, (3β,7α) ;
- Androst-5-en-3β-ol, 7α-bromo-, acétate ;
- Androst-5-en-3β-ol, 7α-bromo.

10. Utilisation cosmétique d'au moins un dérivé de la DHEA de formule (I) selon l'une quelconque des revendications précédentes pour prévenir ou traiter les signes cutanés du vieillissement et/ou le teint terne et/ou les troubles de pigmentation de la peau ou des cheveux et/ou le dessèchement de la peau et/ou l'hyperséborrhée et/ou les imperfections liées à l'hyperséborrhée et/ou les peaux sensibles et/ou les pellicules et/ou la chute des cheveux et/ou la canitie.

11. Utilisation selon la revendication 10, **caractérisée en ce que** les signes cutanés du vieillissement sont choisis parmi : les rides et ridules, l'atrophie cutanée, la perte de fermeté et/ou d'élasticité de la peau, un grain de peau irrégulier avec présence de pores dilatés, la perte d'éclat de la peau et/ou les taches pigmentaires.

12. Composition cosmétique renfermant, dans un milieu physiologiquement acceptable, au moins un dérivé de la DHEA de formule (I) tel que défini dans l'une quelconque des revendications 1 à 9 et au moins un composé choisi parmi : un agent desquamant, un agent hydratant, un agent dépigmentant ou propigmentant, un agent anti-glycation, un inhibiteur de NO-synthase, un inhibiteur de 5α-réductase, un inhibiteur de lysyl et/ou prolyl hydroxylase, un agent stimulant la synthèse de macromolécules dermiques ou épidermiques et/ou empêchant leur dégradation, un agent stimulant la prolifération des fibroblastes et des kératinocytes et/ou la différenciation des kératinocytes, un agent myorelaxant, un composé diminuant l'irritation d'origine neurogène, un agent anti-microbien, un agent tenseur, un agent anti-pollution ou anti-radicalaire, un agent apaisant capable d'inhiber au moins une enzyme choisie parmi les phospholipases A2, les lipoxygénases et/ou les prostaglandines humaines synthétases.

13. Composition cosmétique renfermant, dans un milieu physiologiquement acceptable au moins un dérivé de la DHEA de formule (I) tel que défini dans l'une quelconque des revendications 1 à 9 et au moins un pigment inorganique éventuellement enrobé et/ou au moins un filtre UV choisi parmi :
(a) un dérivé de benzophénone ;
(b) un dérivé de triazine ;
(c) l'acide benzène 1,4 di(3-méthylidènecampho 10-sulfonique), éventuellement sous forme partiellement ou totalement neutralisée ;
(d) un dérivé de l'acide salicylique ;
(e) un dérivé de l'acide cinnamique ;
(f) un dérivé de β,β'-diphénylacrylate liquide ;
(g) un dérivé de l'acide p-aminobenzoïque ;
(h) le 4-méthyl benzylidène camphre ;
(i) l'acide 2-phénylbenzimidazole 5-sulfonique ;
(j) un dérivé de 1,3,5-triazine ;
(k) un extrait de végétal choisi parmi un extrait de *Rosmarinus officinalis*, de *Leontopodium alpinum* et/ou de *Leontopodium stracheyi* ;
(I) une silicone benzotriazole de formule :

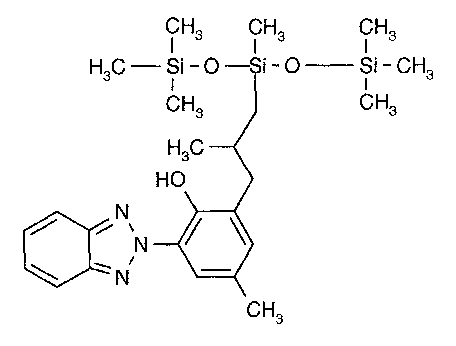

14. Composition selon l'une quelconque des revendications 12 et 13, **caractérisée en ce qu'**elle est adaptée à une application topique sur les matières kératiniques.

15. Composition selon la revendication 14, **caractérisée en ce que** les matières kératiniques sont choisies parmi : la peau, les cheveux, les cils et les ongles.

16. Composition selon l'une quelconque des revendications 12 à 15, **caractérisée en ce qu'**elle renferme de 0,00001% à 10% en poids de dérivé de la DHEA de formule (I), par rapport au poids total de la composition.

17. Composition selon la revendication 16, **caractérisée en ce qu'**elle renferme de 0,001% à 5% en poids de dérivé de la DHEA de formule (I), par rapport au poids total de la composition.

18. Procédé de traitement cosmétique des matières kératiniques comprenant l'application topique sur celles-ci d'une composition renfermant, dans un milieu physiologiquement acceptable, au moins un dérivé de la DHEA de formule (I) tel que défini dans l'une quelconques des revendications 1 à 9, seul ou en association avec au moins un composé tel que défini dans l'une quelconque des revendications 12 et 13.

19. Procédé de traitement cosmétique des signes cutanés du vieillissement et/ou du teint terne et/ou des troubles de pigmentation de la peau ou des cheveux et/ou du dessèchement de la peau et/ou de l'hyperséborrhée et/ou des imperfections liées à l'hyperséborrhée et/ou des peaux sensibles et/ou des pellicules et/ou de la chute des cheveux et/ou de la canitie, comprenant l'application topique sur la peau ou les cheveux d'une composition renfermant, dans un milieu physiologiquement acceptable, au moins un dérivé de la DHEA de formule (I) tel que défini dans l'une quelconques des revendications 1 à 9, seul ou en association avec au moins un composé tel que défini dans l'une quelconque des revendications 12 et 13.

20. Procédé de traitement cosmétique selon la revendication 19, **caractérisée en ce que** les signes cutanés du vieillissement sont choisis parmi : les rides et ridules, l'atrophie cutanée, la perte de fermeté et/ou d'élasticité de la peau, un grain de peau irrégulier avec présence de pores dilatés, la perte d'éclat de la peau et/ou les taches pigmentaires.
